# EUROPEAN PATENT APPLICATION

(11) **EP 2 428 161 A2**
(43) Date of publication of application: **14.03.2012**
(21) Application number: 10188291.8
(22) Date of filing: 20.10.2010
(51) Int. Cl.: A61B 5/06

(54) **Method and system for localising an ingestible element for the functional investigation of the digestive tract**

(30) Priority: 10.09.2010 US 879629
(71) Applicant: ASSISTANCE PUBLIQUE, HOPITAUX DE PARIS, 75004 Paris (FR); UNIVERSITE PARIS DESCARTES, 75270 Paris Cedex 06 (FR)
(72) Inventor: Bouchoucha, Michel, 75017, PARIS (FR)
(74) Representative: Bentz, Jean-Paul

(57) **Abstract**

The present invention relates to a non-invasive method for assessing the digestive motility or transit of a subject, comprising: swallowing a non digestible transmitting element which contains a transmission means transmitting at a given fixed frequency; measuring in series the phase-shift of the frequency transmitted by each of transportable reception means, relative to a reference phase of a reference beacon, to fix the origin of a coordinates system and the coordinates of each transportable reception means in said system, each reception means comprising at least a transceiver, said reception means being distributed around said subject, and said reference beacon being attached to a given fixed location on said subject, measuring then, using said transportable reception means distributed around said subject, the phase shift of the frequency transmitted by said transmission means of said ingestible element; determining by triangulation a position of said ingestible element in said coordinates system.

## Description

The present invention relates to a method of non-invasive ambulatory exploration allowing the assessment of digestive motility and/or transit, and a corresponding system.

Currently the techniques proposed for measuring the digestive motility are invasive, requiring intubation of the patient (manometric and electromyographic techniques).

In order to measure the transit, isotropic or radiopaque markers are used.

These known techniques in particular have the following drawbacks: they cannot be easily applied in everyday clinical practice and require a very significant human and material input;
problems of toxicity are encountered in particular in children, pregnant women;
it is often impossible to correlate the phenomena recorded for studying the motility of an organ with transit measurements.

The purpose of the present invention is to overcome these drawbacks and to propose a method and a system of non-invasive exploration which can, at a low cost, assess the function of each organ involved in digestion (stomach, small intestine, colon), and more particularly simultaneously assess the digestive motility and transit.

It proposes a method of non-invasive exploration for assessing the digestive motility and/or transit of a human or animal subject, characterized in that it consists of:
said subject swallowing an ingestible transmitting element which is non-digestible containing means transmitting at a given fixed frequency;
measuring, at a given time using at least three, preferably four, frequency reception means distributed around said subject's trunk, the phase shift of the frequency transmitted by said transmission means relative to a reference phase;
determining by triangulation on the basis of the three phase-shift measurements the position of said element;
defining, according to the position of said element, a data for the assessment of the digestive motility and/or transit.

The proposed system, comprises:
an ingestible transmitting element which cannot be digested by said subject containing means transmitting at a given fixed frequency;
one fixed cutaneous transmitter used to define the reference of the body for all measurements, and
frequency receiving means comprising at least three, preferably four, frequency receivers intended to be placed around the trunk of said subject, each receiver being able to measure at a given time the phase shift of said transmission frequency relative to a reference phase;
means for processing and analyzing the three, preferably four, phase-shift measurements made by said receivers which are able to determine, by triangulation, the position of said element.

Thus, according to one embodiment, a transmitter circulates in the digestive system, while the data collection takes place in an ambulatory manner using at least three, preferably four, receivers distributed for example on the circumference of the abdominal belt. The basic principle is to measure at a given time the phase shift which is produced between two identical frequencies when the distance from the transmitting source relative to its reference position (corresponding for example to the transmitter in the mouth) is varied. Preferably a high-frequency transmission is chosen because it allows improvement in the precision of the measurements. Using three, preferably four, phase-shift measurements made approximately simultaneously, it is then possible to determine, by triangulation, the position in a three-dimensional reference frame of the transmitter and to deduce a characteristic relating to the digestive motility and/or transit (for example using software including data interpretation). The solution proposed here is:

ambulatory and no longer invasive: only the transmitting element is ingested, but in the same way as a capsule of medicinal product or similar; the receivers are worn by the subject, for example mounted on a standard belt; the patient is free in his movements, his relative position having no influence;

non-toxic: materials exist which can constitute an envelope for the transmitter which are suitable in terms of consistency and non-toxic, for example plastic materials (non-digestible); the transmitting element is for single-use and is eliminated naturally (the risk of cross infection is eliminated); it allows the assessment of the function of an organ involved in digestion: by monitoring the position of the transmitting element, it is possible to measure:

gastric emptying time;

intestinal transit time;

colonic transit time; knowing the weight of the transmitting element, it is possible to measure:

gastric activity

intestinal activity;

colic activity;

the propulsive force of the stomach, the intestine and the colon.

low cost of the proposed technique: the manufacturing costs of the reception element are low: standard miniature electronic components can be used in an impervious envelope; the equipment required by the doctor is limited (belt and analysis software); moreover, in a single examination information is obtained which previously was obtained by different explorations; it should be noted here that the invention could meet, in a simple and inexpensive way, a real requirement of doctors: the number of diagnoses of constipation/diarrhoea was estimated in **1990** at **300,000** by **500** gastroenterologists surveyed (Dorema figures).

Furthermore, information which no other examination could previously give can be provided by the system according to the invention:

measurement of the length of the small intestine; and

measurement of the length of the colon.

According to other embodiment, the number of frequency receivers can be greater than three, preferably four, in order to refine the triangulation and increase the precision of the system and also in order to be able to rule out a measurement which is clearly wrong. Moreover, memory means, which can be common to the receivers, will be used to store in an ambulatory manner the measurements made by the frequency receivers, the analysis and processing of the data then being able to be carried out by subsequently connecting these memory means to a central processing unit, for example a personal computer, equipped with appropriate analysis and processing software. Furthermore, the transmitting element can comprise an integrated and/or induced power supply with, for example, a standby mode and an active mode. In the case of an induced power supply, induction means can be placed on the abdominal circumference, for example on the same belt as that carrying the receivers. Means can also be provided for controlling the transmission at time intervals chosen for the measurements, allowing an appropriate monitoring of the transmitting element.
In the belt all receptors are preferably located in two planes or more.

The present invention will be better understood and other advantages and characteristics will become apparent in light of the following description of an embodiment, which refers to the drawings in which:

FIG. **1** is a schematic view of an embodiment of the system according to the invention, showing a man wearing a belt which is part of the system and having ingested the transmitting element;

FIG. **2** illustrates a step of recovering and processing the data recorded in an ambulatory manner; and

FIG. **3** schematically illustrates an embodiment of electronic components for processing the signals picked up.

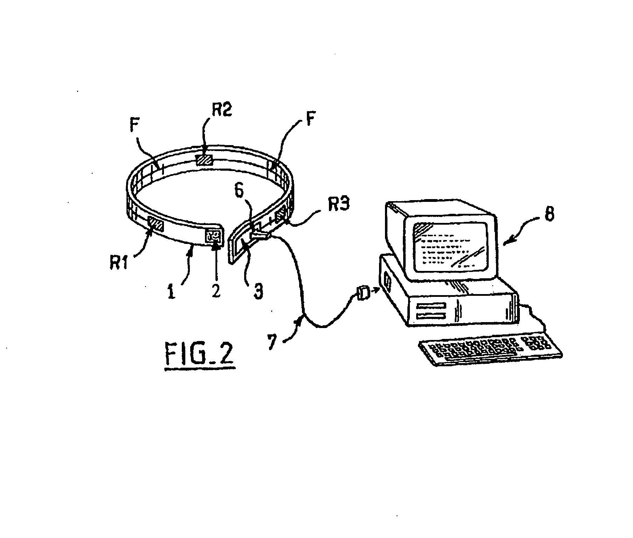

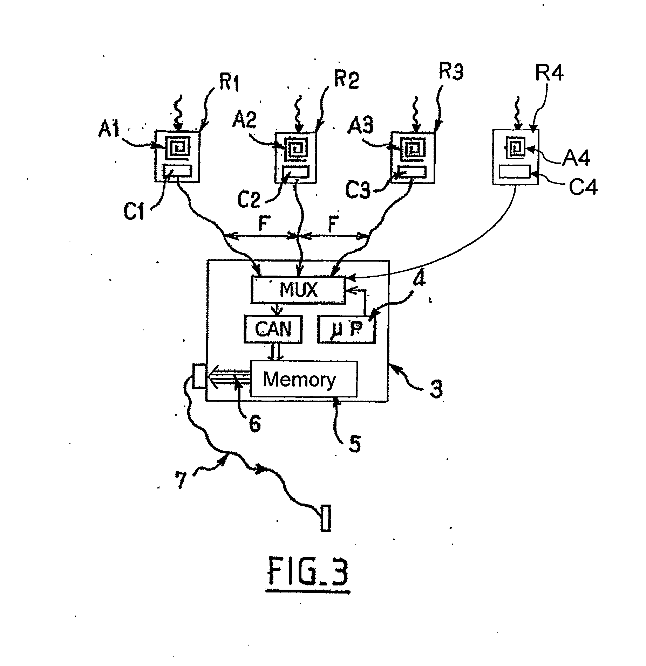

According to the example chosen and represented in the figures, the system comprises a belt **1,** with standard fastening means **2,** on which three frequency receivers **R1, R2** and R3 are arranged, each distributed at a distance from one another on the belt, at approximately the same distance. Each receiver, respectively **R1, R2, R3,** is constituted by a receiving antenna, respectively **A1, A2** and **A3,** which is able to pick up a given high-frequency transmitted by a transmitting element E intended to be ingested by the subject. In each receiver **R1, R2, R3,** an electric circuit C1, C2, C3 is provided which is able to convert the signal picked up by the antenna **A1, A2, A3,** into an electric signal and comprising a local amplifier. The three receivers **R1, R2** and **R3** are connected by cable way (symbolized by F in FIGS. **2** and **3****)** to a buffer card **3** also arranged on the belt **1** (see FIGS. **2** and **3****).** The card **3** comprises means for supplying energy **4,** an MUX multiplexer, an analogue-to-digital converter ADC and memory means 5 which are able to store the measurements made by the three receivers **R1, R2** and **R3** at a given time or given time intervals and an output **6** for a connection cable 7, for example, of USB type. The cable **7** serves to transfer the digital data stored in the card **3** to data analysis and processing means, for example, a personal computer **8** equipped with appropriate software for interpretation of the data transmitted, for display, etc. Each receiver **R1, R2, R3** can be a transceiver. The system also comprises another similar receiver or transceiver **R4,** also comprising an antenna A4 and an electric circuit C4, and acting as a reference beacon. Such reference beacon is not placed on the belt with the other transceivers, but attached to a given fixed location on said subject, e.g. on the sternum. In operation, the reference beacon must remain at a given (fixed) location by any appropriate means (e.g. with glue), whereas the belt **1,** thus receivers **R1, R2, R3,** is removable.

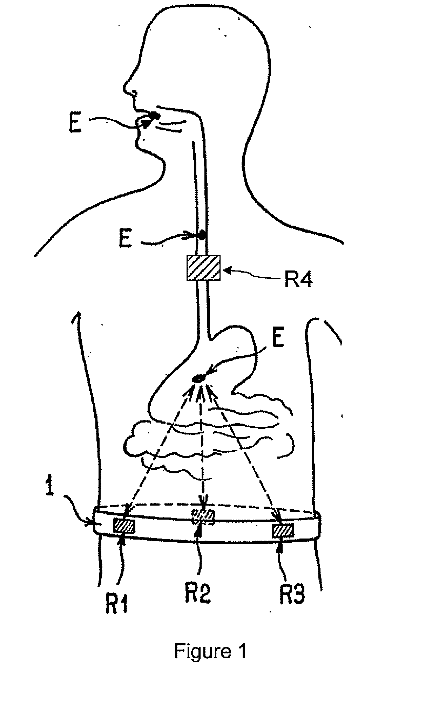

In an embodiment, the system comprises the transmitting element E, represented in three positions in the human digestive system shown schematically in FIG. **1**. Element E comprises means for transmitting at a given frequency. This can be an oscillator driven by a resonator, the output occurring on an antenna for the transmission. The transmitting element E also comprises integrated means for supplying transmitting means with energy, for example a metal oxide type battery and means for controlling said power supply means which allow the change from a standby mode to an active mode. The transmission frequency is preferably chosen to be high, for example **868** MHz, in order to increase the precision of the measurements, as was explained above. It is also chosen among the frequencies permitted in the medical field. The dimensions of the components must be compatible with the fact that the transmitting element is to be ingested. Electronic components of the standard SMC miniature components type can be used. Moreover, these different components are placed in an impervious envelope constituted by a non-digestible material, sufficiently rigid to pass in particular the pylorus at the exit of the stomach, which is non-toxic and non-allergenic. This can be for example an envelope made of plastic material in the form of a gelatin capsule, capsule or similar.

Moreover it is possible to use this system to measure physiological values such as pH, pressure, temperature, etc. For this purpose, the transmitting element will include a corresponding sensor. The transmission of measurements made by this sensor will be transmitted to the receiving means in the form of a modulation of the amplitude of the frequency transmitted by the transmitter corresponding to the amplitude of the signal provided by said sensor.

The system operates in the following way. The belt **1** is fitted around the waist of the subject.
The transmitting element E is placed in the mouth of the subject and then moves naturally in the digestive system, while the subject is free in his movements during the measurements, thanks to the belt 1. At certain selected time intervals, corresponding e.g. to a change in the power supply of the transmitting means from the standby mode to the active mode, the receivers **R1, R2 R3,** and R4 simultaneously pick up the frequency transmitted by the element E. Each signal picked up is collected and stored in the memory card 3. For each quarto of values of signals picked up, a software determines the phase shift relative to a reference position, then determines by triangulation the 3D position of the transmitting element E and, using programmed interpretation instructions, can further provide results regarding the transit time in an organ, a distance covered, a length of a segment of the digestive system and as a function of the weight of the transmitting element it will provide results on the activity of an organ, for example its propulsive force.
In particular, over a given period of time, the position of the transmitting element E is considered to remain globally constant but is subject to slight variations according to a frequency corresponding to natural activity of the organ in which transmitting element E is located. In the fasting state, each digestive segment has a specific activity. For example, this activity has a frequency of 3 cpm (cycles per minute) for the stomach, 12 cpm for the duodenum, 8 cpm for the ileon. Accordingly, triangulation measures enable to measure such natural movement frequency, which value enables to assume in what organ said transmitting element E is located. Before each pick up of the frequency transmitted by the element E, on a first step, the phase-shift of the frequency transmitted by one of said at least three transportable transceivers of belt 1 is measured, relative to a reference phase of the reference beacon and the other transportable transceivers of belt 1. The same is applied for each transceiver of belt 1, in series. Corresponding measures are recorded in a memory, e.g. in the card **3,** and processed to enable calibration of a measuring system. By fixing the position of the reference beacon as the origin of said measuring system, it also enables to determine the coordinates of each transportable transceivers of belt 1 in said system. At each pick up of the frequency transmitted by the element **E,** the measuring system is thus accurately calibrated, enabling to record accurate data, even if the belt has been removed and repositioned between two sets of measurements of the frequency transmitted by the element **E,** or if subject has changed its own position, leading to change of position of belt 1 transceivers. Thus removable belt allows subject to keep having a physical activity even during measurements. Belt 1 is also naturally adaptable to several subjects.
When the examination is considered to be complete, the belt is removed from the subject and the card **3** can be connected at the desired time via the cable **7** to the computer **8,** where the digital data from the card **3** will be able to be analyzed by a data analysis and processing software. The transmitting element E is for single-use, and is eliminated naturally.

In order to allow a better quantization of the transit time of an organ, several transmitting elements can be ingested successively, for example on successive days, in order to produce a multiple measurement in a stable physiological state.

Of course other variant embodiments of the system are possible, in particular, means can be provided for programming the transmission of the transmitting element at selected intervals.

Belt can contain more than three transceivers. In one embodiment, belt 1 contains four transceivers. Preferably, transceivers are not all coplanar. In one embodiment, transceivers on belt 1 are equidistant.

Advantageously, proposed method is independent of feeding.

## Claims

1. A method of non-invasive exploration for assessing the digestive motility or transit of a human or animal subject, comprising:
said subject swallowing an ingestible transmitting element which is non-digestible and contains a transmission means transmitting at a given fixed frequency;
measuring [in series] the phase-shift of the frequency transmitted by each of at least three transportable reception means, relative to a reference phase of a reference beacon, to fix the origin of a coordinates system and the coordinates of each transportable reception means in said system, each reception means comprising at least a transceiver, said reception means being distributed around said subject, and said reference beacon being attached to a given fixed location on said subject,
measuring then, using said transportable reception means distributed around said subject, the phase shift of the frequency transmitted by said transmission means of said ingestible element to obtain at least three phase-shift measurements;
determining by triangulation on the basis of said phase-shift measurements a position of said ingestible element in said coordinates system.

2. The method according to claim 1, further comprising defining, according to the position of said ingestible element, a data for an assessment of the digestive motility and/or transit.

3. The method according to claim 1 or 2, wherein said at least three phase-shift measurements are stored in a memory means.

4. The method according to claim 1 or 2, wherein said reception means are placed around an abdominal belt.

5. The method according to claim 1 or 2, wherein a series of position measurements are made which are spread over time.

6. The method according to claim 1 or 2, wherein a position reference measurement is made when said ingestible element is in the mouth of the subject, before the subj ect swallows it.

7. The method according to claim 1 or 2, wherein a power supply of the transmitting element is triggered at given times and the corresponding phase-shift measurements at each given time are stored in a memory means.

8. The method according to claim 1 or 2, wherein the amplitude of the transmission frequency of the transmission means is modulated as a function of the amplitude of a signal picked up by a sensor included in the transmitting element, said sensor being able to pick up a signal representing a physiological characteristic.

9. The method according to claim 1 or 2, wherein said subject ingests several transmitting elements over a period of time, each transmitting element having a characteristic frequency.

10. A non-invasive exploration system for assessing the digestive motility or transit of a human or animal subject, comprising
an ingestible transmitting elements which cannot be digested by said subject and contains a transmission means transmitting at a given fixed frequency;
receiving means comprising at least three transportable transceivers, each transceiver being able to measure at a given time the phase shift of said transmission frequency relative to a reference phase; and a reference beacon being attached to a given fixed location on said subject;
processing means for fixing said reference beacon position as the origin of a coordinates system and determining the coordinates of each transportable transceivers in said system according to said phase-shift measurements, and for analyzing said at least three phase-shift measurements made by said receivers and determining, by triangulation, the position of said element.

11. The system according to claim 10, wherein it also comprises a memory for storing the phase-shift measurements made by the at least four receivers at a given time.

12. The system according to claim 10, wherein the transmission frequency is a high frequency.

13. The system according to claim 11, wherein the transmitting elements comprises an integrated power supply means.

14. The system according to claim 10, wherein the transmitting element comprises an induced power supply means.

15. The system according to claim 10, wherein the at least four receivers are distributed on a belt which is able to be fixed on said subject.

16. The system according to claim 15, wherein said at least four receivers are not coplanar.

17. The system according to claim 16, wherein the belt also comprises an induction means for the induction of a power supply of said transmitting element.

18. The system according to claim 16, wherein the analysis and processing means include a card comprising means for analogue-to-digital conversion of signals picked up and memory means common to the at least three receivers and arranged on the belt.

19. The system according to claim 11, further comprising a connecting means for connecting a memory means to the processing and analysis means and for transferring data relating to the phase shifts measured.

20. The system according to claim 11, wherein the transmitting element comprises a sensor which is able to pick up a signal representing a physiological characteristic, the amplitude of the frequency transmitted by the transmission means being able to be modulated as a function of the amplitude of the signal picked up by said sensor.

21. The system according to claim 11, comprising several transmitting elements intended to be ingested by said subject over a period of time.
